# EUROPÄISCHE PATENTSCHRIFT

(11) **EP 0 439 767 B1**
(45) Veröffentlichungstag und Bekanntmachung des Hinweises auf die Patenterteilung: **14.02.1996**
(21) Anmeldenummer: 90124316.2
(22) Anmeldetag: 15.12.1990
(51) Int. Cl.: B65H 63/00, G01N 33/36

(54) **Verfahren zur Qualitätsbewertung von Garnen und Einrichtung zur Durchführung des Verfahrens**
Method for evaluating the quality of yarns and apparatus for implementing said method
Méthode pour l'évaluation de la qualité de fils et dispositif pour la mise en oeuvre de cette méthode

(30) Priorität: 26.01.1990 CH 259/90; 08.06.1990 CH 1937/90
(43) Veröffentlichungstag der Anmeldung: 07.08.1991
(73) Patentinhaber: ZELLWEGER LUWA AG, CH-8610 Uster (CH)
(72) Erfinder: Aemmer, Peter F., Dr., CH-8907 Wettswil (CH)

(56) Entgegenhaltungen:
- EP-A- 439 768
- DE-A- 2 820 097
- US-A- 3 577 854
- US-A- 4 184 769

## Beschreibung

Die Erfindung betrifft ein Verfahren zur Qualitätsbewertung von Garnen durch Ermittlung der Garnfehler und deren Klassierung und Zählung zum Zweck der Festlegung der Reinigungsgrenze einer Garnreinigungsanlage.

Bei einem von der Garnfehlerklassieranlage USTER CLASSIMAT (USTER - eingetragenes Warenzeichen der Zellweger Uster AG) bekannten Verfahren dieser Art werden die Garnfehler nach Länge und Querschnitt in insgesamt 23 Klassen für kurze Dickstellen, Doppelfäden, lange Dickstellen und lange Dünnstellen klassiert. Mit Hilfe der USTER KORRELATOR Schablonen werden aus den CLASSIMAT Resultaten unter Berücksichtigung der Anwenderwünsche die Reinigungsparameter (Bezugslänge und Empfindlichkeit) ermittelt. Die entsprechenden Werte für den Fehlerquerschnitt (= Empfindlichkeit) und für die Bezugslänge, das ist die Länge, über die der Mittelwert des Garnquerschnitts gebildet wird, werden am Garnreiniger eingestellt. Obwohl sich dieses Verfahren in der Praxis durchgesetzt und sehr gut bewährt hat, wird aus heutiger Sicht das Hantieren mit Schablonen als umständlich empfunden.

Selbstverständlich weist auch das gereinigte Garn noch Garnfehler auf, und zwar solche, die bei der Reinigereinstellung als tolerierbar eingestuft wurden. Somit besteht im Hinblick auf diese nicht ausgereinigten Garnfehler ein gewisser Qualitätsunterschied zwischen verschiedenen Spulen mit gereinigtem Garn. Ein solcher Qualitätsunterschied besteht aber auch bezüglich anderer Parameter, wie zum Beispiel der Anzahl Spleisse oder Knoten, der Anzahl Nissen, oder des Variationskoeffizienten der Gleichmässigkeit (CV%). Ausserdem besteht auch ein Unterschied bezüglich der beim USTER CLASSIMAT verwendeten Garnfehlerklassen.

In der EP-Patentanmeldung 0 439 768 ist ein Verfahren zur qualitativen Klassierung von elektronisch gereinigtem Garn beschrieben, bei dem durch Vergleich der Querschnittssignale mit zusätzlichen Klassierungsgrenzen, welche enger sind als die jeweiligen Reinigungsgrenzen, eine sogenannte virtuelle Garnreinigung erfolgt.

Aus der DE-A-2820097 ist ein Verfahren zur Qualitätsbestimmung von Garnen und die Einstellung von elektronischen Garnreinigern bekannt, bei dem die Häufigkeit der Garnfehler je Längeneinheit des Garns mit Hilfe einer Modellfunktion berechnet wird. Dabei stützt sich diese Modellfunktion auf Annahmen und Vereinfachungen, die nur in einem engen Bereich die wirklichen Vorgänge genügend genau annähert. Die Leistung eines solchen Verfahrens ist trotz hohem Aufwand deshalb eher beschränkt.

Durch die Erfindung soll nun ein Verfahren zur Qualitätsbewertung von Garnen angegeben werden, mit welchem einerseits die genannte virtuelle Reinigung wirkungsvoll unterstützt werden kann, und welches anderseits verglichen mit dem USTER CLASSIMAT-System leistungsfähiger und flexibler und auch weniger umständlich ist.

Diese Aufgabe wird erfindungsgemäss dadurch gelöst, dass die Einstellparameter Bezugslänge und Empfindlichkeit in Relation zur Anzahl der entsprechenden Reinigereingriffe gesetzt werden und dadurch ein Reinigungsprofil erstellt wird, welches den funktionalen Zusammenhang zwischen der Menge aller theoretisch möglichen Kombinationen der genannten Einstellparameter und den Reinigereingriffen darstellt, und welchem für beliebige Kombinationen von Einstellparametern die zu erwartende Anzahl von Reinigereingriffen entnehmbar ist. Dabei ist die Bezugslänge auf der x-Achse, die Empfindlichkeit auf der y-Achse und die Anzahl der Reinigereingriffe auf der z-Achse eines dreidimensionalen, rechtwinkligen Koordinatensystems aufzutragen. Dann ist das Reinigungsprofil durch eine von den positiven Koordinatenhalbachsen begrenzte, gegen den Ursprung gewölbte Fläche gebildet.

Durch Ermittlung der Reinigungsprofile für gegebene Garne in Kombination mit gegebenen Typen von Garnreinigern ist es möglich, für jede beliebige aller existierenden Kombinationen der Einstellparameter eines bestimmten Reinigertyps eine Prognose der zu erwartenden Schnitthäufigkeit zu erstellen, indem auf der x-yKoordinatenebene der einer Einstellung der Reinigungsparameter entsprechende Punkt ermittelt und auf diesem eine Senkrechte errichtet wird. Die z-Koordinate des Durchstosspunkts dieser Geraden durch das Reinigungsprofil entspricht dann der bei der gegebenen Einstellung zu erwartenden Schnitthäufigkeit.

Die Erfindung betrifft weiter eine Einrichtung zur Durchführung des genannten Verfahrens mit Messköpfen zur Abtastung des Querschnitts eines zu bewertenden Garns, mit einem Steuergerät und mit Auswerteeinheiten, an welche die Messköpfe angeschlossen sind.

Die erfindungsgemässe Einrichtung ist dadurch gekennzeichnet, dass das Steuergerät eine Prozessor-Einheit enthält, in welcher die von den Messköpfen gelieferten und von den Auswerteeinheiten vorverarbeiteten Garnsignale gesammelt, digitalisiert und in Speicherbereichen abgelegt, und die in den Speicherbereichen abgelegten Garnsignale abgearbeitet werden, und dass bei der Abarbeitung für eine Vielzahl von Wertepaaren der Einstellparameter eine virtuelle Reinigung erfolgt, bei welcher die Garnsignale mit einer Mehrzahl von verschiedenen Klassierungsgrenzen verglichen werden, wobei jedes Ueberschreiten einer Klassierungsgrenze eine Registrierung eines virtuellen Reinigereingriffs auslöst. Die Klassierungsgrenzen bilden Stützwerte für das Reinigungsprofil und dessen Darstellung erfolgt in Form einer Grafik.

Im folgenden wird die Erfindung anhand eines Ausführungsbeispiels und der Zeichnungen näher erläutert; es zeigen:
- Fig. 1: ein Diagramm zur Funktionserläuterung mit einem erfindungsgemässen Reinigungsprofil; und
- Fig. 2: eine Gesamtansicht einer Einrichtung zur Durchführung des erfindungsgemässen Verfahrens.

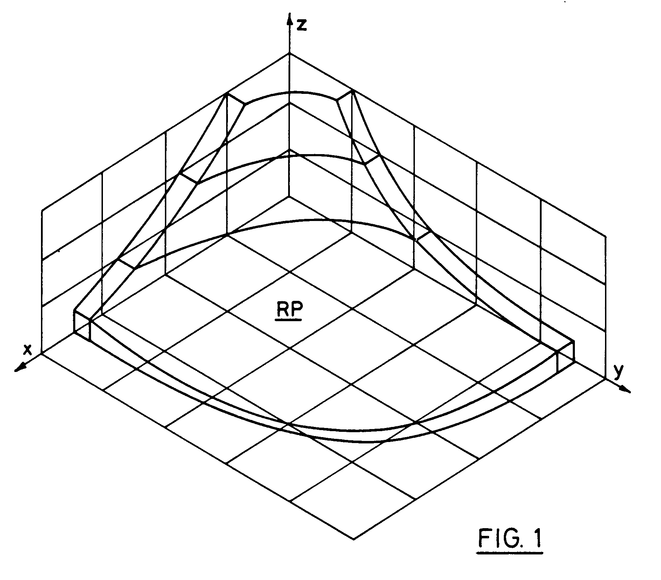

Fig. 1 zeigt ein dreidimensionales rechtwinkliges Koordinatensystem, auf dessen x-Achse die sogenannte Bezugslänge (= Länge, über die der Mittelwert des Garnquerschnitts gebildet wird), auf dessen y-Achse die Empfindlichkeit (= Fehlerquerschnitt) und auf dessen z-Achse die Schnitthäufigkeit (letztere vorzugsweise in einem logarithmischen Massstab) für eine bestimmte Garnreinigungsanlage aufgetragen sind. Die Menge aller Punkte mit positiven Koordinaten des durch diese Koordinatenachsen begrenzten Raumes stellt die Menge aller theoretisch möglichen Kombinationen von Einstellparametern und Reinigerschnitten aller möglichen Garnreinigungsanlagen dar.

Bei einem gegebenen Garn gegebener Länge definiert die Menge aller theoretisch möglichen Kombinationen der Einstellparameter (Bezugslänge, Empfindlichkeit) einer gegebenen Garnreinigungsanlage eine Menge von zu jeder dieser möglichen Kombinationen mit der gegebenen Garnreinigungsanlage zu erwartenden Reinigerschnitten. Die Erfahrung zeigt, dass diese Menge der Reinigerschnitte eine stetige und differenzierbare Funktion der Menge der möglichen Kombinationen der Einstellparameter ist, und daraus folgt, dass sich die Zahl der sich bei jeder möglichen Kombination von Einstellparametern ergebenden Reinigerschnitte in dem dargestellten Koordinatensystem als stetige Fläche darstellen lässt.

Diese in Fig. 1 mit RP bezeichnete und nachfolgend Reinigungsprofil genannte Fläche ist der geometrische Ort der Anzahl aller Reinigerschnitte bei der Reinigung eines gegebenen Garns gegebener Länge auf einer gegebenen Garnreinigungsanlage als Funktion aller beliebigen Kombinationen der Einstellparameter dieser Garnreinigungsanlage. Da aufgrund der Erfahrung für jedes praktisch existierende Garn generell mit abnehmender Bezugslänge und abnehmender Empfindlichkeit eines Garnreinigers die Zahl der zu erwartenden Reinigerschnitte steigt, hat das Reinigungsprofil die in Fig. 1 gezeigte, nach innen gewölbte, oder bezogen auf den Koordinatenursprung, konkave Form.

Die Ermittlung der Reinigungsprofile spezieller Reinigungstypen für gegebene Garne gegebener Länge erfolgt dadurch, dass messtechnisch einzelne Stützwerte bestimmt und aus diesen unter Berücksichtigung der statistischen Unschärfe durch Interpolation und Glättung die für die Darstellung benötigten Zwischenwerte algorithmisch berechnet werden. Die messtechnische Bestimmung der Stützwerte des Reinigungsprofils erfolgt dadurch, dass man einen Garnreiniger so ausbildet, dass er für alle diese Stützwerte eine virtuelle Reinigung der in der CH-Patentanmeldung Nr. 00 259/90-4 beschriebenen Art durchführt. Das heisst also, dass dieser Garnreiniger für alle Kanäle und für alle möglichen Klassierungsgrenzen das Ueberschreiten der jeweiligen Grenzwerte registriert und zählt.

Wenn diese Reinigungsprofile ermittelt sind, dann ist es möglich, für jede beliebige aller existierenden Kombinationen der Einstellparameter eines Reinigertyps eine Prognose der zu erwartenden Schnitthäufigkeiten zu erstellen, indem man auf der x-y-Koordinatenebene den einer bestimmten Einstellung der Reinigungsparameter entsprechenden Punkt ermittelt und in diesem Punkt eine Senkrechte errichtet. Dann entspricht die z-Koordinate des Durchstosspunktes dieser Senkrechten durch die Fläche des Renigungsprofils der bei der gegebenen Einstellung zu erwartenden Schnitthäufigkeit.

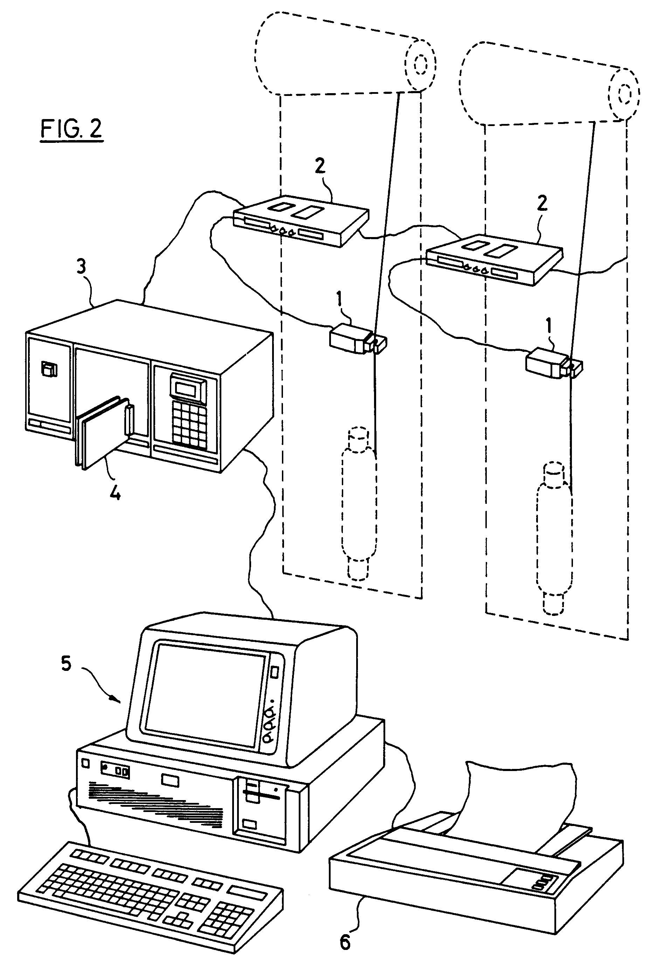

Fig. 2 zeigt eine Gesamtdarstellung einer nach dem erfindungsgemässen Verfahren arbeitenden Garnfehlerklassierungsanlage. Diese besteht darstellungsgemäss aus einer Anzahl von auf einer Spulmaschine angeordneten Messköpfen 1 von der Art, wie sie auch für die elektronische Garnreinigung verwendet werden. Die Messköpfe 1 sind mit Auswerteeinheiten 2 verbunden, welche ihrerseits an ein Steuergerät 3 angeschlossen sind. Dieses Steuergerät entspricht technisch demjenigen der Garnreinigungsanlage USTER POLYMATIC mit dem sogenannten Q-Paket, das ist eine spezialisierte Prozessoreinheit 4, die folgende Funktionen wahrnimmt:
1. Sammlung und Pufferung der von den Messköpfen 1 stammenden Garnsignale:
   - Aufnahme der von der Messköpfen 1 stammenden Analogsignale.
   - Abspeicherung der digitalisierten Garnsignale in Puffer-Speicherbereichen und zwar derart, dass Garnsignale desselben Messkopfs jeweils in einem zusammenhängenden Speicherbereich abgelegt werden, und dass diese Speicherbereiche als "Trommelspeicher" organisiert und verwaltet sind.
2. Abarbeitung der in den genannten Speicherbereichen abgelegten Garnsignale:
   - Abarbeitung eines Speicherbereichs nach dem anderen in zeitlich geraffter Form.
   - Abarbeitung jedes Speicherbereichs derart, dass eine virtuelle Reinigung für eine Vielzahl von Wertepaaren aus Bezugslänge und Empfindlichkeit durchgeführt wird.
   - Wahl der für die virtuelle Reinigung massgebenden Wertepaare derart, dass diese innerhalb eines applikatorisch interessanten Gebietes eines durch die Achsen Bezugslänge/Empfindlichkeit beschriebenen rechtwinkligen Koordinatensystems so zu liegen kommen, dass die Dichte dieser Wertepaare in der Nähe des Ursprungs des Koordinatensystems hoch ist und mit zunehmendem Abstand abnimmt.
   - Wahl eines Koordinatensystems, bei dem eine oder beide Achsen nach einer linearen, einer logarithmischen oder einer annähernd logarithmischen Skala geeicht sind.
   - Abspeicherung der Resultate der virtuellen Reinigung in Pufferspeicherbereichen derart, dass Resultate von Garnsignalen desselben Messkopfs jeweils in einem zusammenhängenden Speicherbereich abgelegt werden, und dass diese Speicherbereiche als "Trommelspeicher" organisiert und verwaltet sind.

Wie sich Fig. 2 weiter entnehmen lässt, ist das Steuergerät 3 mit einem Personal Computer 5 verbunden, an welchen ausserdem ein Drucker 6 angeschlossen ist. Die Verwendung des Personal Computers 5 hat unter anderem dem Vorteil, dass dessen Bildschirm zur Darstellung der Resultate zur Verfügung steht. Für diese Darstellung sind folgende Möglichkeiten vorgesehen:
- Dreidimensionale grafische Darstellung des Reinigungsprofils, die auf dem Bildschirm eventuell durch die Funktionen "Zoom" und "Rotation" ergänzt werden kann.
- Zweidimensionale Darstellung des Reinigungsprofils als kotierte Normalprojektion, und zwar entweder als rechteckförmiges Tabellenschema ähnlich wie die Darstellung beim USTER CLASSIMAT, wobei in den einzelnen Feldern die Stützwerte eingetragen sind, oder in Form von "Höhenkurven" gleicher Schnitthäufigkeit.
- Vorzugsweise wird bei den erwähnten Darstellungsarten auf dem Bildschirm ein Cursor eingeblendet, welcher mittels eines geeigneten Eingabemediums auf der Ebene der x-y-Achsen bewegt werden kann. Der den Koordinaten (Einstellwerten) des Cursors entsprechende Wert der Schnitthäufigkeit wird dann im dreidimensional dargestellten Reinigungsprofil als Durchstosspunkt und/oder in einem Textbalken als Zahlenwert dargestellt.

Dieser Prognosewert der Schnitthäufigkeit zeigt die bei der betreffenden Reinigereinstellung zu erwartenden Anzahl von Reinigerschnitten direkt an und macht somit das bisherige Hantieren mit Schablonen und dergleichen überflüssig.

Wenn auch in Fig. 1 ein rechtwinkliges, kartesisches Koordinatensystem dargestellt ist, so ist diese Darstellung keineswegs als einschränkend zu verstehen, und es können selbstverständlich auch andere Koordinatensysteme verwendet werden.

Auch die mehrfach verwendeten Begriffe Bezugslänge und Empfindlichkeit sind nicht so zu verstehen, dass zur Erstellung des Reinigungsprofils nicht auch andere Parameter verwendet werden könnten. So kann beispielsweise anstatt der Bezugslänge die Fehlerlänge und anstatt der Empfindlichkeit die Querschnittsänderung verwendet werden. Ausserdem kann die Qualitätsbewertung des Garns zusätzlich oder alternativ zur Ermittlung der genannten Garnfehler auch durch Ermittlung der Imperfektionen erfolgen, worunter man die sogenannten "häufigen" Fehler versteht, das sind Dünnstellen, Dickstellen und Nissen, die beim CLASSIMAT-System nicht als Fehler klassiert und die üblicherweise auch nicht ausgereinigt werden. Bezüglich der Imperfektionen wird auf die Publikation USTER News Bulletin, Nr. 26, Nov. 1978, Kapitel: "Die Vertrauensbereiche von Dünnstellen, Dickstellen und Nissen", verwiesen.

## Patentansprüche

1. Verfahren zur Qualitätsbewertung von Garnen durch Ermittlung der Garnfehler und deren Klassierung und Zählung zum Zweck der Festlegung der Reinigungsgrenze einer Garnreinigungsanlage, dadurch gekennzeichnet, dass die Einstellparameter Bezugslänge (x) und Empfindlichkeit (y) in Relation zur Anzahl (z) der entsprechenden Reinigereingriffe gesetzt und je auf der einen Achse eines dreidimensionalen, vorzugsweise kartesischen Koordinatensystems aufgetragen werden und dadurch ein Reinigungsprofil (RP) erstellt wird, welches eine von den positiven Koordinatenhalbachsen begrenzte Fläche bilden, welche den funktionalen Zusammenhang zwischen der Menge aller theoretisch möglichen Kombinationen der genannten Einstellparameter und den Reinigereingriffen darstellt, und welcher für beliebige Kombinationen von Einstellparametern die zu erwartende Anzahl von Reinigereingriffen entnehmbar ist.

2. Verfahren nach Anspruch 1, dadurch gekennzeichnet, dass für die Ermittlung des Reinigungsprofils (RP) einzelne Stützwerte messtechnisch bestimmt und aus diesen die für die Darstellung benötigten Zwischenwerte durch Interpolation berechnet werden.

3. Verfahren nach Anspruch 2, dadurch gekennzeichnet, dass die messtechnische Ermittlung der Stützwerte des Reinigungsprofils (RP) durch eine gleichzeitige virtuelle Reinigung aller Stützwerte eines gegebenen Paars von Einstellparametern erfolgt, wobei die Garnsignale mit einer Mehrzahl von Klassierungsgrenzen verglichen werden, wobei jedes Ueberschreiten einer Klassierungsgrenze eine Registrierung eines virtuellen Reinigereingriffs auslöst und jede Klassierungsgrenze einem Stützwert entspricht.

4. Verfahren nach Anspruch 3, dadurch gekennzeichnet, dass die Darstellung des Reinigungsprofils (RP) in Form einer dreidimensionalen Grafik erfolgt.

5. Verfahren nach Anspruch 3, dadurch gekennzeichnet, dass die Darstellung des Reinigungsprofils zweidimensional, als kotierte Normalprojektion, erfolgt.

6. Verfahren nach Anspruch 5, dadurch gekennzeichnet, dass das Reinigungsprofil als eine mehrere Felder enthaltende Tabelle dargestellt wird, in deren einzelne Felder die Stützwerte eingetragen sind.

7. Verfahren nach Anspruch 5, dadurch gekennzeichnet, dass das Reinigungsprofil in Form von Kurven mit jeweils gleicher Schnitthäufigkeit dargestellt wird.

8. Verfahren nach einem der Ansprüche 1 bis 7, dadurch gekennzeichnet, dass als Parameter zur Erstellung des Reinigungsprofils (RP) die Fehlerlänge und/oder die Querschnittsänderung verwendet werden.

9. Verfahren nach einem der Ansprüche 1 bis 7, dadurch gekennzeichnet, dass zur Qualitätsbewertung des Garns die sogenannten Imperfektionen, das sind die üblicherweise nicht ausgereinigten häufigen Fehler, herangezogen werden.

10. Einrichtung zur Durchführung des Verfahrens nach Anspruch 1, mit Messköpfen (1) zur Abtastung des Querschnitts eines zu bewertenden Garns, mit einem Steuergerät (3) und mit Auswerteeinheiten, an welche die Messköpfe angeschlossen sind, dadurch gekennzeichnet, dass das Steuergerät (3) eine Prozessor-Einheit (4) enthält, in welcher die von den Messköpfen (1) gelieferten und von den Auswerteeinheiten (2) vorverarbeiteten Garnsignale gesammelt, digitalisiert und in Speicherbereichen abgelegt, und die in den Speicherbereichen abgelegten Garnsignale abgearbeitet werden, und dass bei der Abarbeitung für eine Vielzahl von Wertepaaren der Einstellparameter eine virtuelle Reinigung erfolgt, bei welcher die Garnsignale mit einer Mehrzahl von verschiedenen Klassierungsgrenzen verglichen werden, die Stützwerte für das Reinigungsprofil (RP) bilden, dessen Darstellung in Form einer dreidimensionalen Grafik erfolgt und wobei jedes Ueberschreiten einer Klassierungsgrenze eine Registrierung eines virtuellen Reinigereingriffs auslöst.

11. Einrichtung nach Anspruch 10, dadurch gekennzeichnet, dass die Wahl der für die virtuelle Reinigung massgebenden Wertepaare der Einstellparameter so erfolgt, dass diese innerhalb eines applikatorisch interessanten Gebiets eines durch die Achsen Bezugslänge (x) und Empfindlichkeit (y) beschriebenes rechtwinkliges Koordinatensystems so zu liegen kommen, dass die Dichte der Stützwerte in der Nähe des Ursprungs des Koordinatentsystems hoch ist und mit zunehmendem Abstand abnimmt.

12. Einrichtung nach Anspruch 11, dadurch gekennzeichnet, dass auf der dritten Achse (z) des Koordinatensystems die Anzahl der Reinigereingriffe aufgetragen ist, und dass eine oder mehrere der Achsen des Koordinatensystems nach einer linearen, einer logarithmischen oder einer annähernd logarithmischen Skala geeicht sind.

13. Einrichtung nach Anspruch 12, gekennzeichnet durch die auf dem Bildschirm (5) erfolgende Einblendung eines auf der Ebene der x-y-Achsen des Koordinatensystems bewegbaren Cursors und durch die Darstellung des den Koordinaten des Cursors entsprechenden Wertes der Anzahl der zu erwartenden Reinigereingriffe als Durchstosspunkt der auf den Schnittpunkt dieser Koordinaten gefällten Senkrechten durch das Reinigungsprofil (RP).

14. Einrichtung nach Anspruch 13, dadurch gekennzeichnet, dass die Darstellung der Werte für den jeweiligen Durchstosspunkt in einem tabellenartigen Schriftfeld, vorzugsweise in einem Textbalken, erfolgt.

## Claims

1. Method for the quality assessment of yarns by determining the yarn faults and their classification and counting for the purpose of establishing the clearing limit of a yarn clearing system, characterised in that the setting parameters, reference length (x) and sensitivity (y), are set in relation to the number (z) of the corresponding clearer interventions and are each plotted on one axis of a three-dimensional, preferably Cartesian system of coordinates and, by this means, a clearing profile (RP) is set up which forms a surface bounded by the positive coordinate semiaxes, which surface represents the functional correlation between the quantity of all theoretically possible combinations of the said setting parameters and the clearer interventions and which reveals the number of clearer interventions to be expected for any desired combination of setting parameters.

2. Method according to Claim 1, characterised in that individual support values are established by measurement for determining the clearing profile (RP) and, from these, the intermediate values required for the illustration are calculated by interpolation.

3. Method according to Claim 2, characterised in that the determination by measurement of the support values of the clearing profile (RP) takes place by a simultaneous virtual clearing of all support values of a given pair of setting parameters, the yarn signals being compared with a plurality of classification limits, each instance of exceeding a classification limit triggering a registration of a virtual clearer intervention and each classification limit corresponding to a support value.

4. Method according to Claim 3, characterised in that the illustration of the clearing profile (RP) takes place in the form of a three-dimensional graph.

5. Method according to Claim 3, characterised in that the illustration of the clearing profile takes place two-dimensionally as a coded standard projection.

6. Method according to Claim 5, characterised in that the clearing profile is illustrated as a table containing a plurality of squares, in the individual squares of which table the support values are plotted.

7. Method according to Claim 5, characterised in that the clearing profile is illustrated in the form of curves with equal frequency of cuts in each case.

8. Method according to one of Claims 1 to 7, characterised in that the fault length and/or the change in cross-section are used as parameters for setting up the clearing profile (RP).

9. Method according to one of Claims 1 to 7, characterised in that the so-called imperfections, these are the frequent faults generally not cleared out, are used for the quality assessment of the yarn.

10. Device for carrying out the method according to Claim 1, having measuring heads (1) for scanning the cross-section of a yarn to be assessed, having a control device (3) and having evaluation units to which the measuring heads are connected, characterised in that the control device (3) contains a processor unit (4), in which the yarn signals supplied by the measuring heads (1) and preprocessed by the evaluation units (2) are collected, digitalised and stored in store areas, and the yarn signals stored in the store areas are processed, and in that a virtual clearing takes place during processing for a multiplicity of pairs of values of the setting parameters, in which virtual clearing the yarn signals are compared with a plurality of different classification limits which form support values for the clearing profile (RP), the illustration of which takes place in the form of a three-dimensional graph, and in which arrangement each instance of exceeding a classification limit triggers a registration of a virtual clearer intervention.

11. Device according to Claim 10, characterised in that the selection of the pairs of values of the setting parameters which are decisive for the virtual clearing takes place in such a way that these pairs of values come to rest within an area of interest for the application of a right-angled system of coordinates defined by the axes reference length (x) and sensitivity (y) in such a way that the density of the support values is high near to the origin of the system of coordinates and decreases with an increasing distance.

12. Device according to Claim 11, characterised in that the number of clearer interventions is plotted on the third axis (z) of the system of coordinates, and in that one or more of the axes of the system of coordinates is/are calibrated according to a linear, a logarithmic or an approximately logarithmic scale.

13. Device according to Claim 12, characterised by the superimposition on the screen (5) of a cursor, which can be moved on the plane of the x-y axes of the system of coordinates, and by the illustration of the value, corresponding to the coordinates of the cursor, of the number of the clearer interventions to be expected as a point of penetration of the vertical line raised on the intersection of these coordinates through the clearing profile (RP).

14. Device according to Claim 13, characterised in that the illustration of the values for the respective point of penetration takes place in a tabular field of writing, preferably in a bar of text.

## Revendications

1. Procédé pour l'évaluation de la qualité de fils par la détection des défauts de fil et leur classification et comptage pour fixer la limite d'épuration d'une installation d'épuration de fils, caractérisé en ce que les paramètres de réglage longueur de référence (x) et sensibilité (y) sont mis en relation avec le nombre (z) des interventions d'épuration correspondantes et sont portés respectivement sur un axe d'un système de coordonnées tridimensionnel, de préférence cartésien et qu'il est établi ainsi un profil d'épuration (RP) qui forme une surface délimitée par les demi-axes de coordonnée positifs qui représente le rapport fonctionnel entre la quantité de toutes les combinaisons théoriquement possibles des paramètres de réglage précités et les interventions d'épuration, et d'où ressort pour des combinaisons sélectives de paramètres de réglage, le nombre attendu d'interventions d'épuration.

2. Procédé selon la revendication 1, caractérisé en ce qu'on définit par des techniques de mesure pour la détermination du profil d'épuration (RP) différentes valeurs d'appui et qu'on calcule à partir de celles-ci par interpolation les valeurs intermédiaires requises pour la représentation.

3. Procédé selon la revendication 2, caractérisé en ce que la détermination par des techniques de mesure des valeurs d'appui du profil d'épuration (RP) a lieu par une épuration virtuelle simultanée de toutes les valeurs d'appui d'une paire donnée de paramètres de réglage, où les signaux de fil sont comparés à une multitude de limites de classification, où chaque dépassement d'une limite de classification déclenche un enregistrement d'une intervention d'épuration virtuelle et chaque limite de classification correspond à une valeur d'appui.

4. Procédé selon la revendication 3, caractérisé en ce que la représentation du profil d'épuration (RP) a lieu sous la forme d'un graphique tridimensionnel.

5. Procédé selon la revendication 3, caractérisé en ce que la représentation du profil d'épuration a lieu de manière bidimensionnel, comme projection normale cotée.

6. Procédé selon la revendication 5, caractérisé en ce que le profil d'épuration est représenté sous forme de table à plusieurs champs, dans les champs de laquelle sont inscrites les valeurs d'appui.

7. Procédé selon la revendication 5, caractérisé en ce que le profil d'épuration est représenté sous forme de courbes respectivement de même fréquence de coupe.

8. Procédé selon l'une des revendications 1 à 7, caractérisé en ce qu'on utilise comme paramètres pour établir le profil d'épuration (RP) la longueur du défaut et/ou la modification de la section transversale.

9. Procédé selon l'une des revendications 1 à 7, caractérisé en ce qu'on utilise pour l'évaluation de la qualité du fil, les soi-disant imperfections, c'est-à-dire les défauts fréquents habituellement non épurés.

10. Dispositif pour la mise en oeuvre du procédé selon la revendication 1, avec des têtes de mesure (1) pour explorer la section transversale d'un fil à évaluer, avec un appareil de commande (3) et avec des unités d'évaluation auxquelles sont raccordées les têtes de mesure, caractérisé en ce que l'appareil de commande (3) comporte une unité de traitement (4) dans laquelle les signaux de fil livrés par les têtes de mesure (1) et traités préalablement par les unités d'évaluation (2) sont collectés, numérisés et stockés dans des zones mémoire, et les signaux de fil stockés dans les zones mémoire sont traités, et en ce que lors du traitement, pour une multiplicité de paires de valeurs des paramètres de réglage, une épuration virtuelle a lieu où les signaux de fil sont comparés à une pluralité de limites de classification différentes qui constituent des valeurs d'appui pour le profil d'épuration (RP) dont la représentation a lieu sous la forme d'un graphique tridimensionnel, et où chaque dépassement d'une limite de classification déclenche un enregistrement d'une intervention d'épurateur virtuelle.

11. Dispositif selon la revendication 10, caractérisé en ce que les paires de valeurs déterminantes pour l'épuration virtuelle des paramètres de réglage sont choisies de façon que celles-ci se situent à l'intérieur d'un domaine intéressant du point de vue de l'application d'un système de coordonnées rectangulaire décrit par les axes de longueur de référence (x) et de sensibilité (y) de telle sorte que la densité des valeurs d'appui est élevée au voisinage de l'origine du système de coordonnées et diminue au fur et à mesure que l'écart augmente.

12. Dispositif selon la revendication 11, caractérisé en ce qu'il est porté sur le troisième axe (z) du système de coordonnées le nombre des interventions d'épurateur, et en ce qu'un ou plusieurs des axes du système de coordonnées sont calibrés suivant une échelle linéaire, logarithmique ou approximativement logarithmique.

13. Dispositif selon la revendication 12, caractérisé en ce qu'on fait apparaître sur l'écran (5) un curseur déplaçable sur le plan des axes x-y du système de coordonnées et en ce qu'on représente la valeur correspondant aux coordonnées du curseur du nombre des interventions d'épurateur attendu comme point de percée de la ligne verticale dirigée sur le point de coupe de ces coordonnées à travers le profil d'épuration (RP).

14. Dispositif selon la revendication 13, caractérisé en ce que la représentation des valeurs du point de percée respectif a lieu dans un champ d'écriture en forme de table, de préférence dans une barre de texte.
